Europäisches Patentamt

European Patent Office

Office européen des brevets

(19)

(11) Publication number: **0 433 074 B1**

(12) **EUROPEAN PATENT SPECIFICATION**

(45) Date of publication of patent specification: **30.11.94**  (51) Int. Cl.5: **A61K 35/12**

(21) Application number: **90313614.1**

(22) Date of filing: **13.12.90**

(54) **Method for treating cardiac malfunction.**

(30) Priority: **13.12.89 US 450048**

(43) Date of publication of application:
**19.06.91 Bulletin 91/25**

(45) Publication of the grant of the patent:
**30.11.94 Bulletin 94/48**

(84) Designated Contracting States:
**AT BE CH DE DK ES FR GB GR IT LI LU NL SE**

(56) References cited:
**WO-A-88/04175**
**US-A- 4 305 872**

(73) Proprietor: **THE GENERAL HOSPITAL CORPO-RATION**
**Office of Technology Affairs**
**75 Boston Street**
**Boston MA 02114 (US)**

(72) Inventor: **Haupert, Garner, Jr.**
**512 Great Road**
**Littleton, Massachusetts 01460 (US)**

(74) Representative: **Hutchins, Michael Richard et al**
**Graham Watt & Co.**
**London Road**
**Riverhead**
**Sevenoaks, Kent TN13 2BN (GB)**

EP 0 433 074 B1

Rank Xerox (UK) Business Services
(3.10/3.09/3.3.3)

## Description

Background of the Invention

Digitalis, digoxin, ouabain and related substances are cardiac glycosides derived from plants. The main pharmacodynamic property of cardiac glycosides is the ability to increase the force of myocardial contraction in a dose-dependent manner (positive inotropic effect). The most probable explanation for the direct positive inotropic effect is the ability of cardiac glycosides to inhibit membrane-bound $Na^+,K^+$-activated adenosine triphosphatase ($Na^+,K^+$-ATPase) Hoffman, B.F. and J.T. Bigger, Jr., "Digitalis and Allied Cardiac Glycosides" in The Pharmacological Basis of Therapeutics (eds. Goodman and Gilman), p. 732 (1980). The hydrolysis of adenosine triphosphate (ATP) by this enzyme provides the energy for the sodium potassium pump.

Relatively little is known about the endogenous regulation of $Na^+,K^+$-ATPase. Catecholamines (Phillis, J.W., Cell, Tissue and Organ Cultures in Neurobiology, pp. 93-97 (1978); Horwitz, B.A., Fed. Proc., 38:2170-2176 (1979)), thyroid hormone (Smith, T.J. and I.S. Edelman, Fed. Proc., 38:2150-2153 (1979)), aldosterone (Rossier, B.C., et al., Science, 12:483-487 (1987)), linoleic and linolenic acids (Bidard, J.N., et al., Biochem. Biophys. Acta., 769:245 (1984); Tamura, M., et al., J. Biol Chem., 260:9672 (1985); and vanadium (Cantley, L.C., Jr., et al., J. Biol. Chem., 243:7361-7368 (1978)) have all been linked to either direct or indirect effects on enzyme activity.

Many researchers have tried to isolate a specific endogenous inhibitor of plasma membrane $Na^+,K^+$-ATPase similar to digitalis or ouabain, but of mammalian origin, by measuring immunoreactivity in plasma, to the digoxin radioimmunoassay in situations where the inhibitor might be elevated. Klingsmueller et al. found digitalis like immunoreactivity in the urine of $Na^+$-loaded normal human subjects (Klingsmueller, et al., Klin. Wochenschr., 60:1249-1253 (1982)). Graves, S.W., et al. made a similar observation in the plasma of uremic subjects (Graves, S.W., et al., Ann. Intern. Med., 99:604-608 (1983)).

The definitive structure of plasma, urinary or tissue inhibitor of $Na^+,K^+$-ATPase is not known (Haupert, G.T., Jr., in The $Na^+$ $K^+$-Pump, Part B: Cellular Aspects; Skou, J.C., et al., Eds., p. 297-320 (1988)). Furthermore, the degree to which various candidate compounds are truly "digitalis-like" in either structure or function remains controversial, since even those substances characterized in the greatest biochemical detail manifest some differences with the cardiac glycosides digitalis and ouabain. (Carilli, C.T., et al., J. Biol. Chem., 260:1027-1031 (1985); Crabos, M., et al., Eur. J. Biochem., 162:129 (1987); Tamura, M., et al., Biochem., 27:4244-4253 (1988)).

For example, the digitalis-like factor (DLF) isolated by Graves cross-reacts with antidigoxin antibodies (Graves, S.W. , U.S. 4,780,314)). However, DLF has never been shown to be a physiologic inhibitor, as would be expected of an endogenous regulator. By physiologic is meant an inhibitor that has a very high binding affinity for the enzyme; reversibly binds and inhibits; has high specificity for the membrane $Na^+,K^+$-ATPase; and is responsive to relevant stimuli.

Because of their positive inotropic effect, cardiac glycosides (e.g. , digitalis and ouabain) are unrivaled in value for the treatment of heart failure. Cardiac glycosides are most frequently used therapeutically to increase the adequacy of the circulation in patients with congestive heart failure and to slow the ventricular rate in the presence of atrial fibrillation and flutter.

However, cardiac glycosides have narrow therapeutic indices and their use is frequently accompanied by toxic effects that can be severe or lethal. The most important toxic effects, in terms of risk to the patient, are those that involve the heart (e.g., abnormalities of cardiac rhythm and disturbances of atrio-ventricular conduction). Gastrointestinal disorders, neurological effects, anorexia, blurred vision, nausea and vomiting are other common cardiac glycoside-induced reactions.

US-A-4 305872, Lubke et al., Chemie u. Biochemie der Aminosauren, Peptide and Protein I, Thieme Verlag Stuttgart, 1975 and H.-D. Jakube, H. Jeschkeit, Aminosauren, Peptide, Proteine, Verlag Chemie, Weinheim, 1982, all describe polypeptide factors produced by the hypothalamus which are capable of regulating (i.e. stimulating or inhibiting) hormone secretion by the pituitary gland. However, none of these documents describes the physiological $Na^+$ $K^+$-ATPase inhibitor described herein and referred to as hypothalamic inhibitory factor (HIF).

Summary of the Invention

This invention relates to Applicant's finding that Hypothalamic Inhibitory Factor, (HIF), has a positive inotropic effect on cardiac muscle cells. Thus, the invention comprises, in one embodiment, Hypothalamic Inhibitory Factor (HIF), preparable by the method set out in Example I as hereinbefore described, or an analogue thereof, for use in therapy.

HIF does not manifest the same toxicity profile as the cardiac glycosides. Therefore, therapy of cardiac malfunctions with HIF can be accomplished with less risk of toxicity to the patient.

This invention further relates to the findings that HIF can be administered therapeutically to treat cardiac glycoside intoxication, edematous disorders and hypotension. Also, HIF can be used to develop specific therapies to prevent hypertension.

Brief Description of the Drawings

Figure 1 is a graph produced by a phase contrast microscope video motion detector showing the effects of ouabain (upper panel) and HIF (lower panel) on cardiac myocyte contractility.

Figure 2 is a graph plotting the inhibitory effects of HIF (2 units/ml) and ouabain (5 mM) or both on total $^{86}Rb^+$ up take by neonatal rat cardiac myocytes.

Figure 3 is a graph plotting the inhibitory effects of increasing doses of HIF on ouabain-sensitive $^{86}Rb^+$ uptake (sodium pump activity) in myocytes.

Figure 4 is a fluorescence signal showing the effect of HIF (1 unit/ml) on cytosolic free $Ca^{+2}$ content in cultured cardiac myocytes exposed for 30 and 60 minutes.

Detailed Description of the Invention

A physiologic $Na^+,K^+$-ATPase regulator has been isolated from bovine hypothalamus and has been named hypothalamic inhibitory factor (HIF). (Haupert, G.T., Jr., et al., Am. J. Physiol., 247:F919 (1984). A method of isolating HIF is described in greater detail in Example I.

HIF satisfies several essential biochemical criteria for a physiologic regulator of the $Na^+$ pump (Haupert, G.T., Jr. and J.M. Sancho, Proc. Natl. Acad. Sci. USA, 76:4658-4660 (1979)). HIF inhibits purified $Na^+,K^+$-ATPase reversibly and, with high affinity ($K_i$ + 1.4 nM) (Haupert, G.T., Jr., et al., Am. J. Physiol., 247:F919-F924 (1984)). In addition, its effects are specific. (Carilli, C.T., et al., J. Biol. Chem., 260:1027-1031 (1985)).

Purified Hypothalmic Inhibitory Factor (HIF) has now been found to have a positive inotropic effect on cardiac muscle cells (i.e., myocytes), as mentioned above. "Positive inotropic effect" means that the contractility of the cells is enhanced in a dose-dependent manner.

A positive inotropic effect-producing amount of HIF can be administered to a "mammalian host" (e.g., a human) to treat cardiac malfunction (e.g., congestive heart failure, paroxysmal atrial tachycardia, atrial fibrillation and flutter). Administration can be either enteral (i.e., oral) or parenteral (e.g., via intravenous, subcutaneous or intramuscular injection). In addition, as HIF does not manifest the same toxicity profile as the cardiac glycosides, therapy of cardiac malfunctions with HIF can be accomplished with less risk of toxicity to the patient.

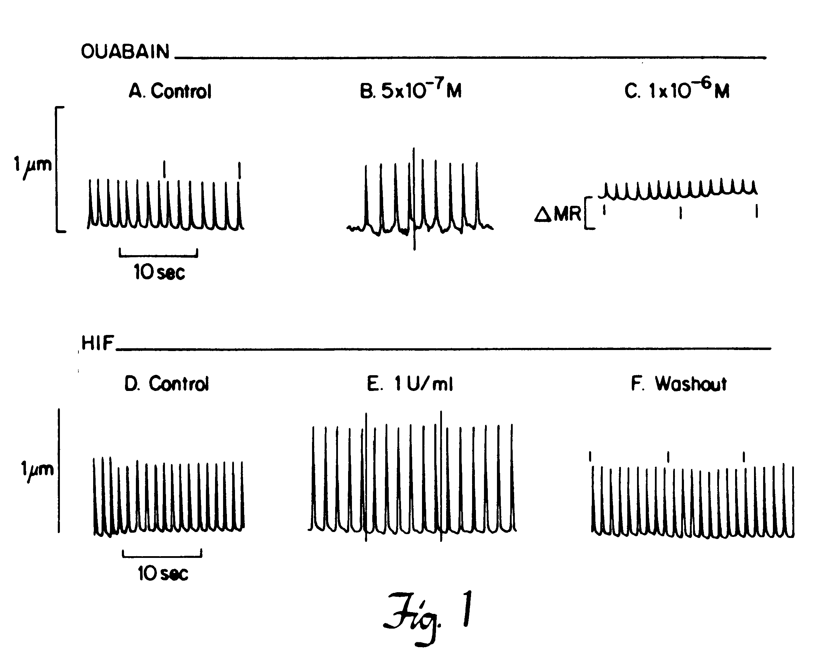

Figure 1 is a graph showing the positive inotropic effects on myocytes exposed to 1 unit/ml of HIF in comparison to 5 X $10^{-7}$ M ouabain; and in comparison to the toxic effects on myocytes exposed to 1 X $10^{-6}$ M ouabain. 1 unit of HIF is the amount necessary to inhibit 1 $\mu$g of highly purified $Na^+, K^+$-ATPase by 50% under standard assay conditions at 37°C. This has been estimated to be about 0.75 pmol HIF (1 unit in 50 microliters which is about equal to 15 nM HIF). (Haupert, G.T., et al., Am. J. Physiol., 247: F919-F924 (1984)). The protocol for the contractility experiments is set forth in Example III C.

In general, the amplitude of contraction (i.e., degree of positive inotropy) was measured in single, beating myocytes as the amplitude of systolic motion (ASM) using a phase contrast video motion detector system. The same detector system was used to measure toxicity, which is evidenced as a decrease in ASM, a change in the position of maximal relaxation (MR), and an increase in beating frequency.

Figure 1B shows that 5 x $10^{-7}$ M ouabain, a maximally inotropic but non-toxic dose in these cells, increases amplitude of systolic motion (ASM) by 41% and caused a decrease in beating frequency compared to the same cells in the control period (Figure 1A). There was no change in the position of maximal relaxation (MR), indicating "therapeutic range" but non-toxic effects.

Figure 1C shows that a 1 uM concentration of ouabain causes a decrease in ASM, an elevation in the position of MR, and an increase in beating frequency, all indicating "toxic range" effects of this higher dose on the contracting myocytes.

Figure 1E shows that 1 unit/ml HIF causes a 37±3% increase in ASM compared to control (Figure 1D), with a decrease in beating frequency and no change in the position of MR. Therefore, 1 unit/ml is within the therapeutic range, but exhibits no toxic effects.

Figure 1F shows the results following a 1 minute "washout", during which the same cell was perfused with HIF-free buffer. ASM and beating frequency returned to control levels indicating rapid reversibility of

EP 0 433 074 B1

the positive inotropic effects caused by HIF.

HIF effects are more readily reversible than those of ouabain, since a 5 minute washout period in ouabain-treated neonatal rat myocytes is required to return enhanced contractility to control levels (Werdan, K., et al., Biochem. Pharmacol., 33:1873-1886 (1984)).

In addition to its use in treating cardiac malfunction, a pharmaceutical composition of HIF can be administered (e.g., enterally or parenterally) to treat patients with serious or life-threatening cardiac glycoside intoxication. Currently, cardiac glycoside intoxication is treated either generally by administering potassium or antiarrythmic drugs to the patient, or specifically by administering antibody fragments to specific cardiac glycoside preparations. Patients with severe toxicity may be unresponsive to general methods of treatment. In addition, although treatment with antibody fragments does neutralize cardiac glycosides in circulation, the antibodies may not effect cardiac glycosides that are bound to cardiac tissue. In addition, because antibodies are proteins, they are administered intravenously and can cause allergic reactions.

In contrast, HIF not only blocks circulating cardiac glycosides from binding to the $Na^+,K^+$-ATPase, but also elutes or "chases" previously bound cardiac glycoside from $Na^+, K^+$-ATPase presumably by competing with or interfering with the cardiac glycoside binding site. "Chase" experiments were performed using an assay system whereby purified $Na^+,K^+$-ATPase is reconstituted into liposomes (Anner, B M. and M. Moosmayer, Biochem. Biophys. Res. Commun., 129:102-108 (1985)). The detailed protocol for these experiments is set forth in Example IV. In genereal, liposomes containing functional $Na^+,K^+$-ATPase molecules were incubated with $^3$H-ouabain which permits measurement of specific ouabain binding to its binding site on the $Na^+,K^+$-ATPase. The liposome-$Na^+,K^+$-ATPase-ouabain complex was then exposed to varying doses of HIF for 10 min at 25°C. The bound $^3$H-ouabain was eluted from the $Na^+,K^+$-ATPase by HIF in a dose-dependent manner, with complete elution of the bound ouabain at an HIF concentration of 0.5 units per 2.5 microliters liposomes.

Thus, HIF is not only able to prevent digitalis compounds from binding to the $Na^+,K^+$-ATPase, but, as shown by these experiments, is able to displace cardiac glycoside already bound to the $Na^+,K^+$-ATPase. Therefore, treatment of cardiac glycoside intoxication with HIF could serve as a highly specific therapy to rapidly reverse the toxic effects on the heart. In addition, as a non-peptide, oral administration of HIF is possible.

HIF can also be administered (e.g., enterally or parenterally) in treating blood pressure abnormalities. Studies have shown that excess of endogenous circulating inhibitor of $Na^+, K^+$-ATPase may be responsible for essential hypertension in some or many patients. (DeWardener, H.E. and G.W. MacGregor, Kidney Int., 18:1-9 (1980)). Presumably, the increased intracellular calcium ion concentration resulting from the binding of an inhibitor to $Na^+, K^+$-ATPase produces blood vessel constriction and hypertension. (Blaustein, M.P., Am. J. Physiol., 232:C165-C173 (1977)).

Experiments were conducted to determine the vasoconstrictive properties of HIF. The protocol for these experiments is described in greater detail in Example V. In general, Sprague-Dawley rats were anesthetized and the abdominal aorta surgically removed. 2 mm vascular rings were attached to a force transducer and bathed in buffer, and tension adjusted to 1.5 g. Tissue viability was documented and vasoconstrictive responses calibrated using known vasoconstrictors such as potassium chloride and norepinephrine. Blood vessels thus prepared were then tested with varing doses of HIF.

HIF produced potent, reversible vasoconstriction of the vessels, and these responses were dose dependent. Vessels remained completely viable after exposure to HIF, documenting absence of toxic effects. Maximum vasoconstrictive responses were similar to those produced by the known vasoconstrictor substances used as standards.

Hypotension, abnormally low blood pressure, can be caused by low cardiac output, inadequate vascular constriction, or both occurring simultaneously. Since HIF has been demonstrated to both increase the strength of cardiac cell contraction and promote blood vessel constriction, its administration in therapeutic amounts would be an effective treatment for hypotension.

HIF can also be used to develop specific therapies to prevent excessive vasoconstriction and resulting hypertension. Such therapies would include: (1) Administering antibodies to HIF for passive immunizations; (2) administering immunogenic forms of HIF for active immunity against hypertension; and (3) administering analogues of HIF which could prevent or modulate binding of native HIF to and action on the vascular cell $Na^+,K^+$-ATPase.

In addition, by potently inhibiting the $Na^+, K^+$-ATPase activity of renal tubular cells and thereby promoting sodium excretion, a pharmaceutical composition of HIF can be used as a natural diuretic, to promote excretion of excess salt and water by the kidneys in patients suffering from such common clinical conditions as congestive heart failure, cirrhosis of the liver, and nephrotic syndrome. Because of the

4

specific inhibitory effect that HIF has on $Na^+,K^+$-ATPase, diuretic therapy with HIF can be accomplished without the side effects (e.g., impotence, rashes, blood lipid abnormalities) which commonly occur with existing diuretic drugs.

This invention is illustrated further by the following examples.

## EXEMPLIFICATION

### I. Preparation of HIF

The $Na^+,K^+$-ATPase inhibitor was prepared from bovine hypothalamus as previously described (Carilli, C.T., et al., J. Biol. Chem., 260:1027-1031 (1985)). Hypothalami collected fresh and frozen immediately on dry ice were thawed, homogenized and extracted in methanol:water (4:1, v:v). Methanol was removed by flash evaporation, and lipids removed by extraction of the remaining aqueous phase with petroleum ether and chloroform. Initial separation of HIF was carried out using lipophilic gel chromatography (Carilli, C.T., et al., J. Biol. Chem., 260:1027-1031 (1985)). Further purification was accomplished using successive cation and anion exchange chromatographies. Approximately 100 units HIF from the lipophilic gel chromatographies was dissolved in 10 ml doubly distilled water ($ddH_2O$), applied to a small column containing 10 ml of sulfonic acid cation exchange resin in the protonated form (Amersham, IR 120), and eluted with $ddH_2O$ at 1 ml/minute. The effluent was collected, lyophilized, the residue taken up in 1 ml $ddH_2O$ and applied to a column containing 1.5 ml QAE Sephadex (Pharmacia) in the hydroxyl form. The column was eluted successively with one bed volume $ddH_2O$) and one bed volume 10 mM acetic acid (the latter used to recover HIF absorbed to the anion exchanger presumably resulting from interaction with the carboxylate group on HIF (Carilli, C.T., et al., J. Biol. Chem., 260:1027-1031 (1985)). Acetic acid was removed by lyophilization, and the residue resuspended and assayed for specific $Na^+,K^+$-ATPase inhibitory activity (HIF) in a coupled-enzyme assay (Haupert, G.T., et al., Am. J. Physiol., 247:F919-F924 (1984)) and a human erythrocyte $^{86}Rb^+$ uptake assay (Carilli, C.T., et al., J. Biol. Chem., 260:1027-1031 (1985)) to determine the concentration of HIF activity. The cation exchange step successfully removed small amounts of gamma amino benzoic acid, serine, threonine and glutamic acid, and the anion exchange step, trace amounts of lactate, all of which had been detected by mass spectroscopic analysis of active fractions following the lipophilic gel chromatographies. Recovery of HIF was quantitative following the ion-exchange steps. HIF following the above procedures is free of vanadate (emission spectroscopy) , $NH_4$ ion (reductive amination, Sigma kit 170-A), and free fatty acids and lysophospholipids (gas chromatography-mass spectroscopy) which have been shown to interfere in pertinent bioassays.

### II. Preparation of Cardiac Cells

Myocardial cells were isolated from ventrical fragments of the hearts of 1-day old Sprague-Dawley rats by serial trypsinizations in a $Ca^{2+}$ and $Mg^{2+}$-free Hanks Buffered Salt Solution (HBSS) as previously described (Yagev, S., et al., In Vitro, 20:893-898 (1984)). Trypsinized cells were decanted into HamF10 medium containing 20% serum and antibiotics and centrifuged at 1000 r.p.m. for 10 minutes. The cell pellet was resuspended in HamF10 medium containing fetal calf serum and 10% horse serum with 0.1% penicillin-streptomycin, and diluted to a concentration of $5 \times 10^5$ cells/ml. For measurements of cytosolic free calcium concentration ($[Ca^{2+}]i$), the cells were plated on rectangular glass coverslips (13 x 30 mm) and for measurements of cell contractility, plated on circular glass coverslips (12 mm), and both types of coverslips placed inside petri dishes. For measurements of $^{86}Rb^+$ uptake cells were plated in petri dishes (1 - 1.5 x $10^6$ cells/35mm dish). All cultures were incubated in humidified 5% $CO_2$, 95% air at 37°C. Confluent monolayers in which an estimated 80% of cells exhibited spontaneous synchronous contractions developed by three days, at which time experiments were performed.

### III. Physiologic Effects of HIF on Cardiac Cells

#### A. $^{86}Rb^+$ Influx Measurements

$Na^+$ pump activity was estimated in the cultured cardiac cells as the difference in $^{86}Rb^+$ uptake observed in the absence and presence of 5 mM ouabain, following the method of Panet et al (Panet, R., et al., J. Memb. Biol., 70:165-169 (1982)). To insure saturation binding, myocytes were preincubated for 20 minutes with HIF (2 unit/ml) or ouabain (5 mM) or both prior to addition of $^{86}Rb^+$ to run the 10 minute flux. Myocyte monolayers were washed with HEPES buffer solution (final concentrations, mM: NaCl 150, RbCl 5, HEPES-Tris 10 (pH7.0) , CaCl$_2$ 1, MgCl$_2$ 5, glucose 10), and the uptake initiated by covering over with 0.5 ml of same solution pre-warmed to 37°C and containing 2 uCi $^{86}$RbCl. Incubations were continued for up to 10 minutes (period of linear uptake of the isotope) (Heller, M., et al., Biochem. Biophys. Acta, 939:595-602 (1988)), and the uptake terminated by aspiration of the reaction mixture followed by two rapid rinses with 3 ml ice-cold MgCl$_2$ (125 mM) and two with 5 ml ice-cold NaCl (165 mM). The cells were lysed with 0.6 ml of 0.1 N NaOH containing 0.1% (w/v) sodium dodecylsulfate, and the radioactivity counted in Instagel (Packard) scintillation medium.

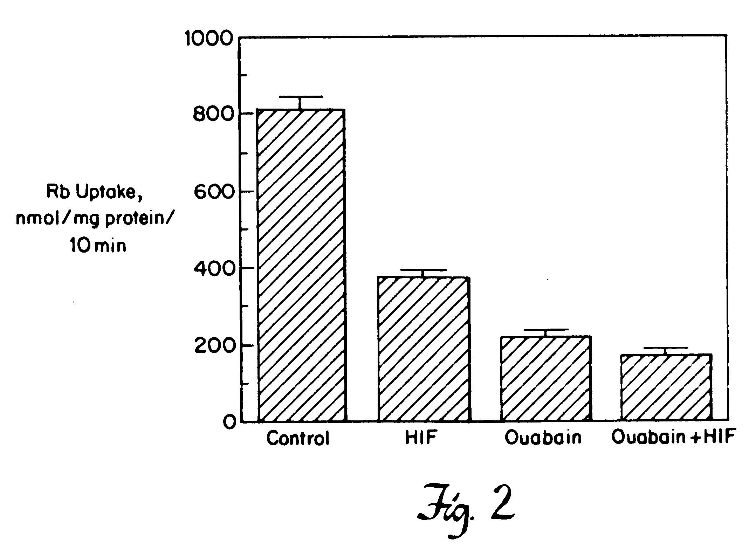

Figure 2 illustrates the inhibitory effects of HIF (2 units/ml) and ouabain (5 mM) or both on total $^{86}$Rb$^+$ uptake by neonatal rat cardiac myocytes. Na$^+$ pump mediated uptake (600 nmol/mg protein/10 min) is calculated as the difference in total uptake, and uptake in the presence of 5 mM ouabain.

Ouabain (5 mM) decreased uptake from control levels of 810 nmol/mg protein to 210 nmol/mg (74%), while HIF (2 units/ml) decreased the uptake from control levels to 377 nmol/mg (54%). The combination of ouabain plus HIF was not additive, indicating that HIF inhibitory effects are specific for K$^+$ transport through the Na$^+$,K$^+$-ATPase, as has been previously shown for human erythrocytes (Carille, C.T., et al., J. Biol. Chem., 260:1027-1031 (1985)) and renal tubular cells (Cantiello, H.F., et al., Am. J. Physiol., 255:F574-F580 (1988)). HIF (2 unit/ml) thus inhibited ouabain-sensitive K$^+$ transport in myocytes by 74%.

The concentration dependence of HIF inhibition was determined by preincubating cells with HIF at 0.5 units/ml, 0.8 units/ml, 1 unit/ml, 2 units/ml and 4 units/ml concentration for 20 min. $^{86}$RbCl (2 uCi/well) was then added to run the flux.

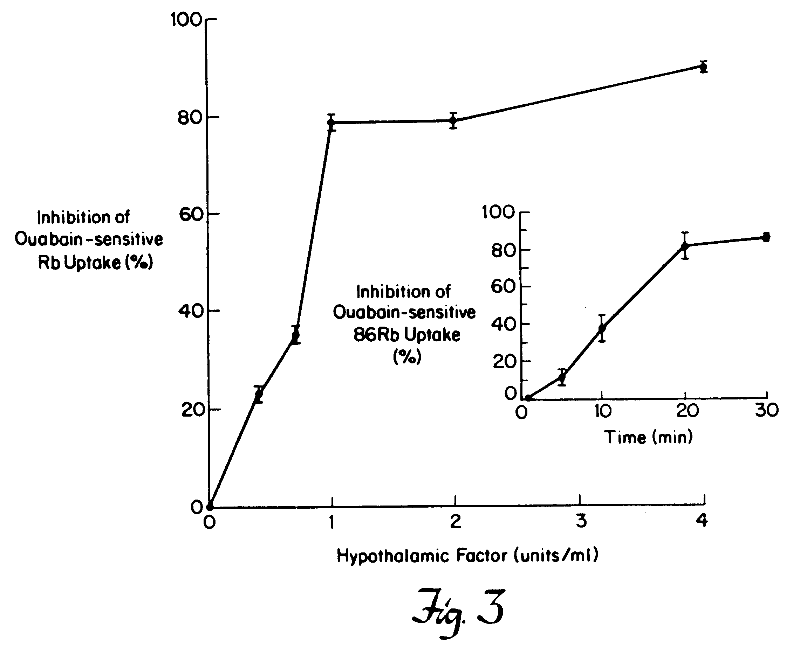

Figure 3 shows the concentration dependence of HIF inhibition of ouabain-sensitive RB$^+$ uptake (a specific measure of Na$^+$,K$^+$-ATPase activity) in neonatal rat cardiac myocytes. Values are mean ± SEM for n = 4 determinations at each concentration. HIF-mediated Na$^+$ pump inhibition is dose-dependent and related to the amount of time that the myocytes are exposed to the inhibitor. Ninety percent of Na$^+$ pump activity in the neonatal rat myocytes is inhibited by the maximal dose of HIF (4 units/ml). Figure 3 (inset) illustrates the inhibition of active K$^+$ transport as a function of the time that myocytes are exposed to HIF (2 units/ml). Maximal inhibitory effects (i.e., inhibition of myocyte Na$^+$,K$^+$-ATPase activity by about 90%) required 20-30 minutes preincubation with HIF.

However, significant pump inhibition also occurred after shorter exposures to HIF. The ID$_{50}$ for pump inhibition in neonatal rat myocytes occurs at an HIF concentration of about 0.5 units/ml. This is approximately 30 fold less than that for cultured porcine renal tubular cells (Cantiello, H.F., et al., Am. J. Physiol., 255:F574-F580 (1988)), therefore suggesting that neonatal rat myocytes have a relatively high affinity for HIF.

B. Measurement of Cytosolic Free Ca$^{2+}$ ([Ca$^{2+}$])

Changes in [Ca$^{2+}$] i were detected using the fluorescent probe, fura-2 (Grynkiewicz, G., et al., J. Biol. Chem., 260:3440-3450 (1985)). Rectangular glass coverslips with attached myocytes were placed in buffered salt solution (BSS, containing in mM: NaCl 140, KCl 5, CaCl$_2$ 1, MgCl$_2$ 1, glucose 10, Na$_2$HPO$_4$ 1, Hepes-Tris 10 (pH 7.4) to which was added 5 uM fura-2/AM, and incubated for 1 hour in humidified 5% Co$_2$-95% air at 37°C. Additional loading medium was added and incubation continued for 15 minutes to complete the hydrolysis of fura-2/AM. The cells were washed and incubated an additional 30 minutes in BSS. Coverslips with loaded myocytes were inserted into a thermostrated (37°C) cuvette containing 2 ml BSS and various additions of HIF or ouabain as indicated. The fluorescence was continuously recorded using a PTI DeltaScan 1 spectrofluorometer. Dual excitation wavelengths alternated rapidly (60 Hz) between 340 nm and 380 nm, emission wavelength 505 nm. Values of [Ca$^{2+}$]i were calculated from the ratio R = F$_{340}$/F$_{380}$ using the formula: [Ca$^{2+}$]i = K$_d$ B (R - R$_{min}$)/(R$_{max}$ -R), where K$_d$ is 225 nM. R$_{max}$ and R$_{min}$ were determined in separate experiments using digitonin to equilibrate [Ca$^{2+}$]i with ambient [Ca$^{2+}$] (R$_{max}$), and addition of MnCl$_2$ (0.1 mM) and EGTA (1 mM) (R$_{min}$). Background autofluorescence was measured in unloaded cells and substracted from all measurements.

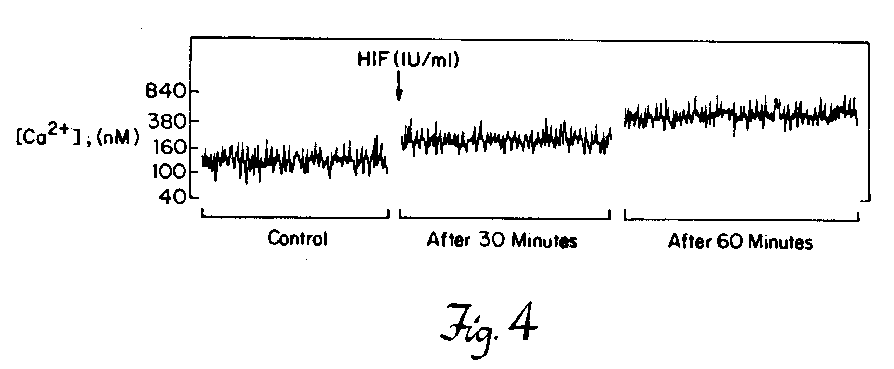

Figure 4 shows the effect of HIF (1 unit/ml) on cytosolic free calcium ion ([Ca$^{2+}$]i) content in cultured cardiac myocytes. Fluorescence of fura-2 loaded myocytes incubated in Buffered Salt Solution (BSS) was recorded continuously. Control value (138 nM) is for cells in BSS prior to addition of HIF (arrow). Recordings are shown after 30 min (250 nM) and 60 min (432 nM) exposure to HIF. Cytosolic free Ca$^{2+}$ concentrations increased from a baseline of 138 nM to 250 nM and 432 nM after 30 and 60 minutes exposure to HIF, respectively.

The onset of change in [Ca$^{2+}$]i caused by HIF occurred within 15 minutes, reached a new steady state concentration by 60 minutes, and remained stable at this level for at least 2 hours.

The biochemical events underlying cardiac glycoside toxicity and the narrow therapeutic index characteristic of these drugs is incompletely understood, although excessive intracellular free $Ca^{2+}$ secondary to persistently elevated intracellular $Na^+$ associated with tonic pump inhibition has been postulated as having a central role (Tsien, R.W. and B.U. Carpenter, Fed. Proc. Fed. Am. Soc. Exp. Biol., 37:2127-2131 (1978)).

Table 1 shows increases in steady-state $[Ca^{2+}]i$ induced by various doses of HIF. A dose-response relationship was found with 0.5 unit/ml HIF increasing $[Ca^{2+}]i$ in the myocytes to a level (303 ± 15 nM) similar to that caused by 1 uM ouabain (287 ± 15 nM) under the same experimental conditions. HIF (1 unit/ml) raised $[Ca^{2+}]i$ to a level significantly greater than that induced by 1 uM ouabain.

TABLE 1: Changes in steady-state cytosolic free $Ca^{2+}$ concentrations ($[Ca^{2+}]i$) in cardiac myocytes treated with ouabain or various concentrations of HIF*

| Condition | Control | Ouabain $10^{-6}$ M | HIF (units/ml) 0.25 | 0.5 | 1.0 |
|---|---|---|---|---|---|
| $[Ca^{2+}]i$ (nM) | 138±3 | 287±15 | 197±9 | 303±15 | 432±18 |

*Values are mean ± SEM; n = 3 determinations at each concentration

While 1 uM ouabain raises the intracellular concentration of $Ca^{2+}$ to 287±15 nM, and causes clear toxicity (Figure 1C), 1 unit/ml of HIF raises the intracellular concentration of $Ca^{2+}$ in the same cells to a much higher level, 432 ± 18 nM, but is accompanied by a stable, maximal inotropic effect with no sign of toxicity (Figure 1E).

C. Measurements of Myocyte Contractility

Contractility, determined as amplitude of systolic cell motion (ASM), and beating frequency were measured in individual cells using a phase contrast microscope video motion detector system according to the method of Barry, et al. (Barry, W.H., et al., Circ. Res., 56:231-241 (1985)). A glass coverslip with attached cultured myocytes was placed in a chamber provided with inlet and exit ports for medium perfusion. The chamber was enclosed in a Lucite box at 37°C and placed on the stage of an inverted phase contrast microscope. The cells were covered with 1 ml medium containing HIF or ouabain. During continuous perfusion, medium bathing a cell in the center of a coverslip exchanged with a time constant of 15 seconds at a flow rate of 0.96 ml/minutes. Image was magnified using a 40x objective, and motion monitored by a low-light-level TV camera attached to the microscope and calibrated with 262 raster lines. The motion detector monitors a selected raster line segment and provides new position data every 16 msec for an image border of a microsphere within the cell layer moving along the raster line. The analog voltage output from the motion detector is filtered at low pass filter and calibrated to indicate actual um of motion, and the derivative is obtained electronically and recorded as velocity of motion in um/sec. Rate, amplitude and velocity of contraction remained stable for several hours during control perfusions. The changes in contractility induced by ouabain or HIF were calculated in comparison with the contractility of the same cells before addition of ouabain or HIF.

Table 2 summarizes changes in ASM and beating frequency as a function of various doses of HIF. Increasing concentrations of HIF caused progressive increase in ASM and decrease in beating rate. Maximal increase in ASM occurred at an HIF concentration of about 0.5 units/ml (39±6%), a level equal to the maximal, non-toxic dose of ouabain (5 x $10^{-7}$ M).

```
TABLE 2:   Effects on amplitude of systolic motion
           (ASM) and beating rate in cardiac myocytes
           by various concentrations of HIF, and
           ouabain (Ou)*
```

| | HIF (units/ml) | | | | | Ou (M) |
|---|---|---|---|---|---|---|
| | 0.2 | 0.25 | 0.33 | 0.5 | 1.0 | $5 \times 10^{-7}$ |
| ASM, % increase | $15 \pm 2$ | $22 \pm 1$ | $32 \pm 9$ | $39 \pm 6$ | $37 \pm 3$ | $41 \pm 3$ |
| Rate, % decrease | $12 \pm 1$ | $15 \pm 1$ | $25 \pm 7$ | $40 \pm 2$ | $49 \pm 6$ | $23 \pm 2$ |

*Values are mean $\pm$ SEM; n=3 for each concentration

IV. HIF ability to displace cardiac glycosides from their binding site on the $Na^+, K^+$-ATPase

"Chase" experiments were performed using an assay system whereby purified $Na^+,K^+$-ATPase is reconstituted into phosphatidylcholine liposomes. ATP-filled liposomes containing dispersed, randomly oriented $Na^+,K^+$-ATPase molecules were prepared by the cholate-dialysis method according to Anner (Anner, B.M. and M. Moosmayer, Biophys. Res. Coommun. 129:102-108 (1985)). Using this miniaturized, two-sided test system Applicant has also shown that $Na^+,K^+$-ATPase inhibition by a single dose of 0.1 U HIF (approximately 75 fmol), and the membrane permeation of 1.0 U HIF (approximately 750 fmol) became measurable, and that an estimation of the minimal number of HIF molecules per unit could be made. Despite testing of numerous substances including other proposed endogenous $Na^+,K^+$-ATPase inhibitors, HIF is the only compound (besides the cardiac glycosides themselves) tested thus far in the purified system that displays such striking transport inhibition.

For "chase" experiments liposomes containing functional $Na^+,K^+$-ATPase molecules were incubated for 10 min at 25°C with $^3$H-ouabain (10 uM), following which HIF was added in various doses and the incubation continued for 10 min at 25°C. Reactions were quenched by addition of 125 ul ice-cold stop solution, and the samples applied and eluted through (0°C) a 20-cm Sephadex G-50 medium column which permits separation of bound from free $^3$H-ouabain. Bound $^3$H-ouabain was eluted from the $Na^+,K^+$-ATPase by HIF in a dose-dependent manner as shown in Table 3.

Table 3: Elution of $^3$H-ouabain bound to 2.5 ul $Na^+,K^+$-ATPase liposomes by various doses of HIF.

| | -HF | +HF (units/2.5 ul liposomes) | | |
|---|---|---|---|---|
| | | 0.125 | 0.25 | 0.5 |
| $^3$H-ouabain bound (cpm) | 2902 | 178 | 111 | 45 |
| $^3$H-ouabain eluted (%) | - | 94.0 | 96.2 | 98.5 |

V. Vasoconstrictive properties of the hypothalamic $Na^+,K^+$-ATPase inhibitor

Sprague-Dawley rats weighing 250-350 grams were anesthetized with pentobarbital intraperitoneally, the abdominal aorta rapidly excised and dissected free of all loose connective tissue in cold Krebs-Henseleit bicarbonate buffer of the following composition, mM: NaCl, 118.3; KCl, 4.7; $MgSo_4$, 1.2; $KH_2PO_4$, 1.2; $CaCl_2$, 2.5; $NaHCO_3$, 25.0; Na-EDTA, 0.016; and glucose, 11.1. 2-3 mm (length) vascular rings were excised, attached to a force transducer and bathed in a water jacketed (37° C) organ chamber containing 5 ml of the above buffer gassed with 95% $O_2$ and 5% $CO_2$. Isotonic force measurements were obtained with a Grass force displacement transducer attached to a Grass 79D polygraph DC amplifier, and recorded with a two-channel Kipp Zonen recorder. Calibration studies revealed that aortic rings placed under 1.5 g tension generated a maximal contractile response after KCl depolarization, and all rings were therefore equilibrated under 1.5 g tension prior to starting experiments. Tissue viability for individual experiments was documented using known vasoconstrictors such as potassium chloride and norepinephrine. Blood vessels thus prepared were then tested with varing doses of HIF, and the magnitude of response compared with KCl-induced contractions. HIF produced potent, reversible vasoconstrictions of the vessels, and these responses were dose dependent as shown in Table 4.

Table 4: Effects on vascular tension in rat abdominal aortic rings by various concentrations of HIF, and KCl, shown as % increase above resting tension of 1.5 g

Change in Tension in Rat Aorta

| | HF (units/ml) | | | | KCl (mM) |
|---|---|---|---|---|---|
| | 0.1 | 0.1 | 0.4 | 0.8 | 20 |
| % increase | 2 | 5 | 12 | 26 | 20 |

Vessels remained completely viable after exposure to HIF as judged by preservation of KCl responses following washout of HIF; documenting absence of toxic effects. Maximum vasoconstrictive responses were similar to those produced by the membrane depolarizing dose of KCl. These studies confirm that HIF is a potent vasoconstrictive substance, compatible with its proposed role in regulation of vascular tone and potential role in the pathogenesis of hypertensive disease.

**Claims**

1. Hypothalamic Inhibitory Factor (HIF), preparable by the method set out in Example I as hereinbefore described, or an analogue thereof, for use in therapy.

2. Hypothalamic Inhibitory Factor (HIF), preparable by the method set out in Example I as hereinbefore described, or an analogue thereof, at a therapeutically effective concentration of 0.1-1.0 unit/ml [0.075-0.75nM] in a therapeutically acceptable carrier, for use in therapy.

3. Hypothalamic Inhibitory Factor (HIF) according to claim 1 or claim 2, wherein the therapy comprises producing a positive inotropic effect in a a mammalian host and/or treating a cardiac malfunction (such as congestive heart failure, paroxysmal atrial tachycardia or atrial fibrillation).

4. Hypothalamic Inhibitory Factor (HIF) according to claim 1 or claim 2, wherein the therapy comprises the treatment of cardiac glycoside intoxication.

5. Hypothalamic Inhibitory Factor (HIF) according to claim 1 or claim 2 wherein the therapy comprises the treatment of an edematous disorder such as congestive heart failure, cirrhosis of the liver and nephrotic syndrome.

6. Hypothalamic Inhibitory Factor (HIF) according to claim 1 or claim 2 wherein the therapy comprises the treatment of hypotension.

7. Hypothalamic Inhibitory Factor (HIF) according to claim 1 or claim 2 wherein the therapy comprises the treatment of hypertension through active immunization with an immunogenic form of HIF.

8. The use of Hypothalamic Inhibitory Factor (HIF) preparable by the method set out in Example I as hereinbefore described, or an analogue thereof, for the manufacture of a medicament for a use in therapy, e.g. in the therapy as defined in any one of claims 3-7.

9. The use of an analogue derived from Hypothalamic Inhibitory Factor (HIF), the HIF from which the analogue is derived being preparable by the method set out in Example I as hereinbefore described, for the manufacture of a medicament for treating hypertension, for example by preventing or modulating the binding of native HIF.

10. The use of an antibody of Hypothalamic Inhibitory Factor (HIF) preparable by the method set out in Example I as hereinbefore described, or an analogue thereof, for the manufacture of a medicament for the treatment of hypertension by passive immunisation.

**Patentansprüche**

1. Hypothalamus-Hemmfaktor (HIF) herstellbar nach dem in Beispiel I dargelegten Verfahren, wie hier zuvor beschrieben, oder ein Analoges davon, zum therapeutischen Einsatz.

2. Hypothalamus-Hemmfaktor (HIF) herstellbar nach dem in Beispiel I dargelegten Verfahren, wie hier zuvor beschrieben, oder ein Analoges davon, in therapeutisch wirksamer Konzentration von 0,1 - 1,0 Einheiten/ml (0,075 - 0,75 nM) in einem therapeutisch verträglichen Träger zum therapeutischen Einsatz.

3. Hypothalamus-Hemmfaktor (HIF) gemäß Anspruch 1 oder 2, worin die Therapie umfaßt die Erzeugung eines positiven inotropen Effekts in einem Mammalia- Wirt und/oder die Behandlung einer Herzfehlfunktion (wie beispielsweise kongestive Herzinsuffizienz, paroxysmale arterielle Tachykardie oder arterielle Fibrillation) .

4. Hypothalamus-Hemmfaktor (HIF) gemäß Anspruch 1 oder 2, wobei die Therapie die Behandlung der glykosidischen Intoxikation des Herzens umfaßt.

5. Hypothalamus-Hemmfaktor (HIF) gemäß Anspruch 1 oder 2, wobei die Therapie die Behandlung ödematöser Störungen wie kongestive Herzinsuffizienz, Leberzirrhose und nephrotisches Syndrom umfaßt.

6. Hypothalamus-Hemmfaktor (HIF) gemäß Anspruch 1 oder 2, wobei die Therapie die Behandlung von Hypertonie umfaßt.

7. Hypothalamus-Hemmfaktor (HIF) gemäß Anspruch 1 oder 2, worin die Therapie die Behandlung von Hypertonie durch aktive Immunisierung mit einer immunogenen Form des HIF umfaßt.

8. Die Verwendung des Hypothalamus-Hemmfaktor (HIF) herstellbar nach dem in Beispiel I dargelegten Verfahren, wie hier zuvor beschrieben, oder eines Analogen davon, zur Herstellung eines Medikamentes für den therapeutischen Einsatz, zum Beispiel bei der in einem der Ansprüchen 3 - 7 beschriebenen Therapie.

9. Die Verwendung eines Analogen abgeleitet vom Hypothalamus-Hemmfaktor (HIF), wobei der HIF, aus dem das Analoge abgeleitet ist, herstellbar ist nach dem in Beispiel I dargelegten Verfahren, wie hier zuvor beschrieben, zur Herstellung eines Medikamentes zur Behandlung der Hypertonie , zum Beispiel durch Verhindern oder Modulieren der Bindung des nativen HIF.

10. Die Verwendung eines Antikörpers des Hypothalamus-Hemmfaktors (HIF), herstellbar nach dem in Beispiel I dargelegten Verfahren, wie hier zuvor beschrieben, oder Analoge davon, zur Herstellung eines Medikamentes zur Behandlung der Hypertonie durch passive Immunisierung.

**Revendications**

1. Facteur d'Inhibition Hypothalamique (HIF), qui peut être préparé par la méthode exposée dans l'Exemple I, comme cela a été décrit précédemment, ou un analogue de celui-ci, pour une utilisation en thérapie.

2. Facteur d'Inhibition Hypothalamique (HIF), qui peut être préparé par la méthode exposée dans l'Exemple I, comme cela a été décrit précédemment, ou un analogue de celui-ci, à une concentration thérapeutiquement efficace de 0,1-1,0 unité/ml [0,075-0,75 nM] dans un vecteur thérapeutiquement acceptable, pour une utilisation en thérapie.

3. Facteur d'Inhibition Hypothalamique (HIF), selon la revendication 1 ou la revendication 2, pour lequel la thérapie comprend la production d'un effet inotrope positif, chez un hôte mammifère et/ou le traitement d'une malfonction cardiaque (telle qu'une défaillance cardiaque congestive, une tachycardie atriale paroxystique ou une fibrillation atriale).

4. Facteur d'Inhibition Hypothalamique (HIF), selon la revendication 1 ou la revendication 2, pour lequel la thérapie comprend le traitement de l'intoxication cardiaque aux glycosides.

5. Facteur d'Inhibition Hypothalamique (HIF), selon la revendication 1 ou la revendication 2, pour lequel la thérapie comprend le traitement d'un désordre oedémateux tel qu'une défaillance cardiaque congestive, une cirrhose du foie et un syndrome néphrotique.

6. Facteur d'Inhibition Hypothalamique (HIF), selon la revendication 1 ou la revendication 2, pour lequel la thérapie comprend le traitement de l'hypotension.

7. Facteur d'Inhibition Hypothalamique (HIF), selon la revendication 1 ou la revendication 2, pour lequel la thérapie comprend le traitement de l'hypertension par une immunisation active, avec une forme immunogène de HIF.

8. L'utilisation du Facteur d'Inhibition Hypothalamique (HIF), qui peut être préparé par la méthode exposée dans l'Exemple I, comme cela a été décrit précédemment, ou d'un analogue de celui-ci, pour la fabrication d'un médicament, pour une utilisation en thérapie, par exemple, dans la thérapie telle que définie dans l'une quelconque des revendications 3-7.

9. L'utilisation d'un analoque dérivé du Facteur d'Inhibition Hypothalamique (HIF), le HIF à partir duquel l'analogue est dérivé pouvant être préparé par la méthode exposée dans l'Exemple I, comme cela a été décrit précédemment, pour la fabrication d'un médicament, pour le traitement de l'hypertension, par exemple par l'empêchement ou la modulation de la liaison de l'HIF natif.

10. L'utilisation d'un anticorps du Facteur d'Inhibition Hypothalamique (HIF), qui peut être préparé par la méthode exposée dans l'Exemple I, comme cela a été décrit précédemment, ou d'un analogue de celui-ci, pour la fabrication d'un médicament, pour le traitement de l'hypertension par une immunisation passive.

Fig. 1

*Fig. 2*

EP 0 433 074 B1

Fig. 3

15

*Fig. 4*